# EUROPEAN PATENT APPLICATION

(11) **EP 4 292 540 A1**
(43) Date of publication of application: **20.12.2023**
(21) Application number: 22752621.7
(22) Date of filing: 28.01.2022
(51) Int. Cl.: A61B 10/00, G16H 50/20, A61B 5/372

(54) **SYMPTOM EVALUATION DEVICE AND SYMPTOM EVALUATION PROGRAM**

(30) Priority: 12.02.2021 JP 2021020806
(71) Applicant: Keio University, Tokyo, 108-8345 (JP)
(72) Inventor: MITSUKURA Yasue, Yokohama-shi, Kanagawa 223-8522 (JP); KISHIMOTO Taishiro, Tokyo 160-8582 (JP); TAZAWA Yuki, Tokyo 160-8582 (JP)
(74) Representative: Grünecker Patent- und Rechtsanwälte PartG mbB
(86) International application number: PCT/JP2022/003452
(87) International publication number: WO 2022/172792

(57) **Abstract**

[Object] Objectively determine a symptom relating to a mental disorder independently of a determiner's subjectivity.

[Solution] The symptom determination apparatus 20 according to the present embodiment includes the EEG feature detection section 212 and the symptom determination section 214. The EEG feature detection section 212 detects one or a plurality of frequency components of an EEG signal in a certain brain area of a subject. The symptom determination section 214 determines a depressive symptom in the subject on the basis of intensity of the one or plurality of frequency components of the EEG signal detected by the EEG feature detection section 212.

## Description

### [Technical Field]

The present invention relates to a symptom determination apparatus and a program for determining a symptom.

### [Background Art]

Determiners, such as physicians, conventionally take the lead in determining symptoms relating to mental disorders including depression. For example, a determiner has a conversation with a subject and determines a symptom relating to a mental disorder on the basis of, for example, the subject's condition during the conversation and how the subject responds.

PTL 1 discloses an example of a technique for assisting such a determiner. In a technique disclosed in PTL 1, a server used by a determiner and a terminal used by a subject are communicably connected to each other, and the determiner is assisted in taking the lead in determining a symptom in the subject, who is at a distant location.

### [Citation List]

### [Patent Literature]

[PTL 1]
Japanese Unexamined Patent Application Publication No. 2016-066317

### [Summary of Invention]

### [Technical Problem]

When a determiner takes the lead in determining a symptom as described above, however, a result of the determination inevitably reflects the determiner's subjectivity. The result of a determination, therefore, can be undesirably different depending on a determiner.

The present invention has been conceived in view of these circumstances. An object of the present invention is to objectively determine a symptom relating to a mental disorder independently of a determiner's subjectivity.

### [Solution to Problem]

In order to achieve the object described above, A symptom determination apparatus comprising:
electroencephalography signal detection means for detecting one or a plurality of frequency components of an electroencephalography signal in a certain brain area of a subject; and
depressive symptom determination means for determining a depressive symptom in the subject on a basis of intensity of the one or plurality of frequency components of the electroencephalography signal detected by the electroencephalography signal detection means.

### [Advantageous Effects of Invention]

According to the present invention, a symptom relating to a mental disorder can be objectively determined independently of a determiner's subjectivity.

### [Brief Description of Drawings]

[Fig. 1] is a block diagram illustrating the overall configuration of a symptom determination system S according to the present embodiment.
[Fig. 2]is a block diagram illustrating an example of the configuration of a EEG apparatus 10.
[Fig. 3] is a block diagram illustrating an example of the configuration of a symptom determination apparatus 20.
[Fig. 4] is a flowchart illustrating how the EEG apparatus 10 performs the measurement process.
[Fig. 5] is a flowchart illustrating how the symptom determination apparatus 20 performs a process for generating reference data.
[Fig. 6] is a flowchart illustrating how the symptom determination apparatus 20 performs a process for determining a symptom.
[Fig. 7] includes graphs illustrating a result of comparison of EEG features between normal persons and patients with depression in this example.
[Fig. 8] includes graphs obtained by enlarging the result of comparison of EEG signals between the normal persons and the patients with depression in this example.
[Fig. 9] is a graph illustrating the result of comparison of EEG features between the normal persons and the patients with depression in this example using relative values.

### [Description of Embodiments]

An example of an embodiment of the present invention will be described hereinafter with reference to the accompanying drawings.

### [System Configuration]

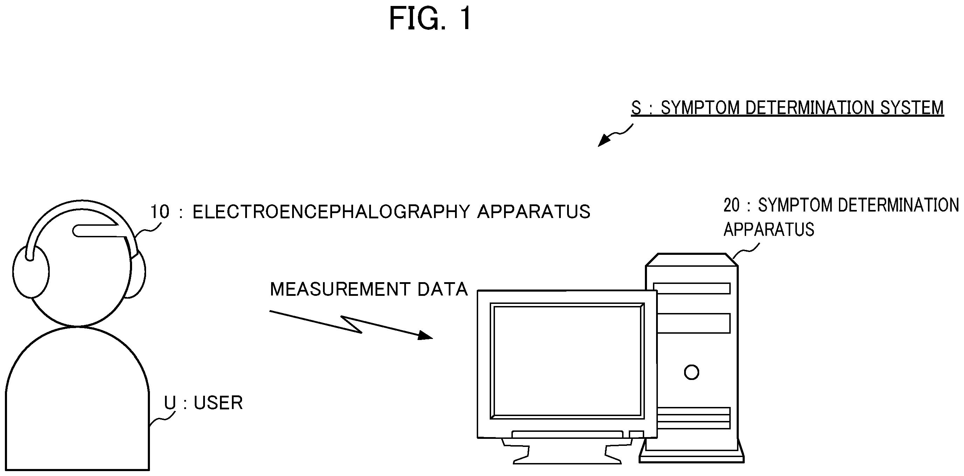

Fig. 1 is a block diagram illustrating the overall configuration of a symptom determination system S according to the present embodiment. As illustrated in Fig. 1, the symptom determination system S includes an electroencephalography (EEG) apparatus 10 and a symptom determination apparatus 20. Fig. 1 also illustrates a user U (i.e., a subject for whom symptom is to be determined) to be subjected to a process performed by the symptom determination system S.

The EEG apparatus 10 and the symptom determination apparatus 20 are communicably connected to each other. Communication between these apparatuses may be performed in compliance with any communication method, and the communication method is not particularly limited. The communication connection may be wired connection or wireless connection. Furthermore, the communication between the apparatuses may be performed directly or over a network including a relay apparatus. In this case, the network is achieved, for example, by a LAN (Local Area Network), the Internet, a mobile phone network, or a network based on a combination of these.

The symptom determination system S objectively determines a symptom relating to a mental disorder exhibited by the user U, who is a subject, independently of a determiner's subjectivity. As a result of a series of pilot studies on a determination of a symptom relating to a mental disorder, the inventor of the present invention found a correlation between symptoms of mental disorders and EEG signals and conceived that such a symptom of a mental disorder could be determined on the basis of an EEG signal, which led to the present invention. In the present embodiment, therefore, it is assumed, as an example for description, that the symptom determination system S determines a symptom relating to depression, which is one of illnesses accompanying a mental disorder. Here, the determination of a symptom relating to depression includes a determination whether the user U is suffering from depression and, if the user U is suffering from depression, a determination of a level of severity of a depressive symptom.

Depression, however, is just an example of illnesses accompanying a mental disorder, and the use of depression as an example is not intended to limit an applicable range of the present invention to depression.

The EEG apparatus 10 generates data (hereinafter referred to as "measurement data") corresponding to the user U's EEG signal through a process such as measurement of variation in electric potential at the user U's head (i.e., an EEG signal in a certain brain area of the user U). The EEG apparatus 10 is implemented as a headset electroencephalograph including a pair of electrodes or multiple electrodes for measuring the user U's EEG signal, each of the electrodes coming into electrical contact with a certain part of the user U. The EEG apparatus 10 generates measurement data by performing a process such as measurement of the user U's EEG signal with each of these electrodes. The EEG apparatus 10 then transmits the generated measurement data to the symptom determination apparatus 20.

The symptom determination apparatus 20 determines the user U's symptom of depression on the basis of the measurement data generated by the EEG apparatus 10. The symptom determination apparatus 20 is achieved, for example, by a personal computer or a server apparatus.

As a specific process, the symptom determination apparatus 20 detects one or a plurality of frequency components of an EEG signal in a certain brain area of the user U, who is the subject. The symptom determination apparatus 20 also determines a depressive symptom of the user U, who is the subject, on the basis of intensity of the detected one or plurality of frequency components of the EEG signal.

The EEG apparatus 10 thus measures an EEG signal in a certain brain area of the user U, who is the subject. The symptom determination apparatus 20 also determines depressive symptom in the subject on the basis of an objective indicator, namely the EEG signal in the certain brain area of the subject.

With the symptom determination system S according to the present embodiment, therefore, a symptom relating to a mental disorder can be objectively determined independently of a determiner's subjectivity.

In addition, since the symptom determination system S objectively determines a symptom relating to a mental disorder, a general problem of the related art can be solved. For example, the above-described problem that a result of a determination inevitably reflects a determiner's subjectivity when the determiner takes the lead in determining a symptom can be solved.

Next, the configuration of EEG apparatus 10 and the symptom determination apparatus 20 and the like for achieving such a process will be described in more detail. A user (corresponds to the user U in Fig. 1) who is subjected to the process performed by the symptom determination system S will be hereinafter referred to as a "subject" as necessary in order to clarify the description. A user (not illustrated) who uses the symptom determination system S in order to determine a symptom for the subject, on the other hand, will be referred to as a "determiner" as necessary.

### [Configuration of EEG Apparatus]

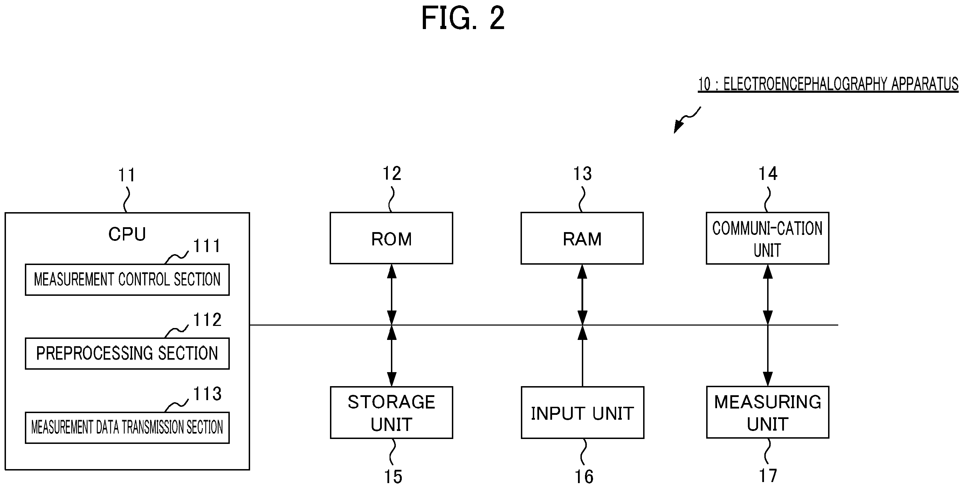

Next, the configuration of the EEG apparatus 10 will be described with reference to Fig. 2. Fig. 2 is a block diagram illustrating an example of the configuration of the EEG apparatus 10.

As illustrated in Fig. 2, the EEG apparatus 10 includes a CPU (Central Processing Unit) 11, a ROM (Read Only Memory) 12, a RAM (Random Access Memory) 13, a communication unit 14, a storage unit 15, an input unit 16, and a measuring unit 17. These components are connected by using a signal line and transmit and receive a signal to and from each other.

The CPU 11 executes various processing (for example, described later a measurement process) according to programs that are recorded in the ROM 12, or programs that are loaded from the storage unit 15 to the RAM 13.

The RAM 13 also stores data and the like necessary for the CPU 11 to execute the various processing, as appropriate.

The communication unit 14 controls communication between the processor 11 and another apparatus (for example, the symptom determination apparatus 20).

The storage unit 15 is configured by DRAM (Dynamic Random Access Memory) or the like, and stores various data managed by each server.

The input unit 815 is configured by various buttons and the like, and inputs a variety of information in accordance with instruction operations by the user.

The measuring unit 17 measures variation in electric potential at the subject's head (here, a suspected patient or a data provider for the symptom determination system S) as the subject's EEG signal. In the present embodiment, the measuring unit 17 is assumed to measure a unipolar EEG signal through reference electrode measurement, which is an example of a measurement method. In this case, one of a pair of electrodes included in the measuring unit 17 is brought into contact with a position where electric potential is close to 0 (e.g., the subject's earlobe) and used as a reference electrode. The other electrode is brought into contact with a certain position on the subject's head (e.g., a position corresponding to Fp1 in the left prefrontal cortex as defined by the International 10-20 system) and used as an exploring electrode. The measuring unit 17 measures variation in a potential difference between the reference electrode and the exploring electrode at a certain sampling frequency (e.g., 512 [Hz]) as an EEG signal in a certain area of the subject's brain.

As described above, the EEG apparatus 10 has a shape of a headset, and the pair of electrodes included in the measuring unit 17 is disposed at positions (e.g., a position on the subject's earlobe and a position on a part corresponding to Fp1) suitable for the measurement when the subject wears the EEG apparatus 10. When an electroencephalograph having a common shape, such as one that looks like an electrode net that covers a subject's head, is used, the subject feels pressure, and tension-induced noise might be undesirably caused in an EEG signal. Since the EEG apparatus 10 has a shape of a headset, on the other hand, the subject does not feel pressure, and the measurement can be performed while suppressing tension in the subject. With the EEG apparatus 10, therefore, occurrence of noise due to tension in the subject can be suppressed, and the measurement can be accurately performed.

In the EEG apparatus 10, these components operate together to achieve the "measurement process".

The measurement process is a process where a subject's EEG signal is measured and certain preprocessing and the like are performed on the measured EEG signal.

When the measurement process is performed, a measurement control section 111, a preprocessing section 112, and a measurement data transmission section 113 function in the CPU 11 as illustrated in Fig. 2.

Although not particularly referred to in the following description, these functional blocks communicate, as necessary, data necessary to achieve the process at appropriate times.

The measurement control section 111 controls, on the basis of an operation command from the subject or the determiner received by the input unit 16 (or command information from the subject or the determiner received through the communication unit 14), the measurement of the subject's EEG signal performed by the measuring unit 17. For example, the measurement control section 111 controls start and end times of the measurement performed by the measuring unit 17, a sampling period of the measurement, and the like. The measurement control section 111 then outputs the subject's EEG signal obtained as a result of the measurement performed by the measuring unit 17 to the preprocessing section 112.

Although the measurement of an EEG signal may be performed in any scene, it is assumed in the present embodiment that the subject's EEG signal is measured while the determiner and the subject have a conversation corresponding to the Hamilton Rating Scale for Depression (HAM-D). Such a conversation, however, need not necessarily take place, and a general conversation such as chatting may be made, instead.

A reason why the measurement is performed during a conversation is that a patient with depression might fall asleep when at rest and, in this case, the patient's EEG signal during sleep is measured. In the present embodiment, therefore, the measurement is performed during a conversation, and an EEG signal while a patient with depression is moderately aroused (i.e., in an ordinary condition) can be measured. In addition, in the present embodiment, the measurement can be performed while removing noise caused by body movement and blinking during a conversation by performing noise removal in the preprocessing, which will be described later, in real-time at certain time intervals.

This, however, is just a preferred example, and an EEG signal may be measured in other scenes. The measurement may be performed for any length of time, but the measurement may last, for example, tens of minutes.

The preprocessing section 112 generates measurement data by preprocessing an EEG signal input from the measurement control section 111. More specifically, the preprocessing section 112 extracts, using a bandpass filter, which is not illustrated, only a frequency component in a certain frequency band (e.g., 1 to 30 [Hz]) from the EEG signal input from the measurement control section 111. Next, the preprocessing section 112 sets a threshold based on a median absolute deviation for the extracted frequency component of the EEG signal at certain time intervals (e.g., 1 second). The preprocessing section 112 then removes outliers exceeding the threshold as mixed noise. The preprocessing section 112 thus generates measurement data by extracting a frequency component in a certain frequency band and removing mixed noise as the preprocessing. The preprocessing section 112 then outputs the generated measurement data to the measurement data transmission section 113.

The measurement data transmission section 113 transmits the EEG data input from the preprocessing section 112 to the symptom determination apparatus 20. The transmission may be performed in real-time as the measurement control section 111 generates EEG data, or the generated EEG data may be stored in the storage unit 15, and after the measurement ends, the EEG data stored in the storage unit 15 may be collectively transmitted at once.

### [Configuration of Symptom Determination Apparatus]

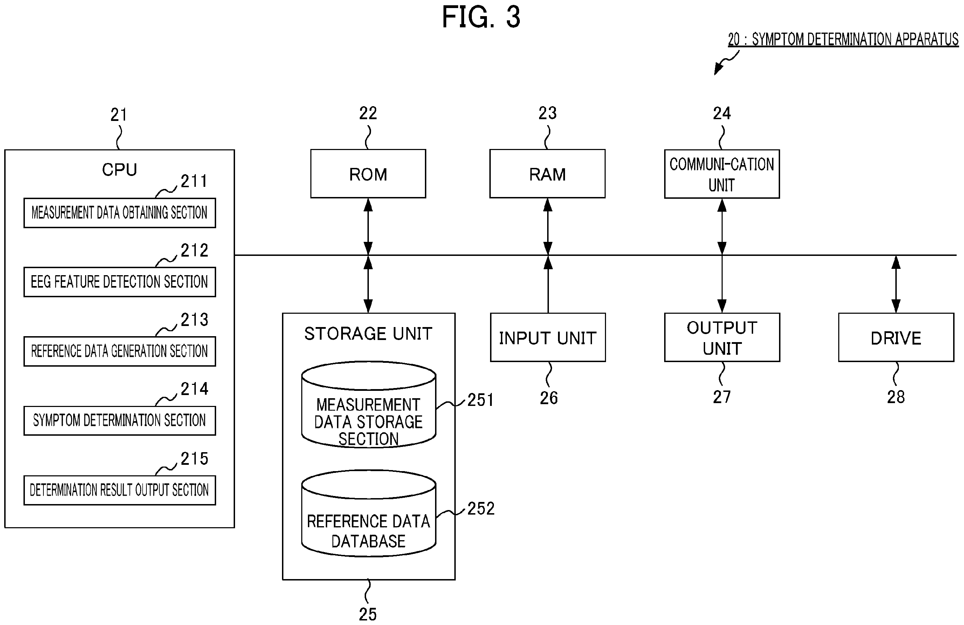

Next, the configuration of the symptom determination apparatus 20 will be described with reference to Fig. 3. Fig. 3 is a block diagram illustrating an example of the configuration of the symptom determination apparatus 20.

As illustrated in Fig. 3, the symptom determination apparatus 20 includes a CPU 21, a ROM 22, a RAM 23, a communication unit 24, a storage unit 25, an input unit 26, an output unit 27, and a drive 28. These components are connected by using a signal line and transmit and receive a signal to and from each other.

The CPU 21 executes various processing (for example, described later a process for generating reference data and a process for determining a symptom) according to programs that are recorded in the ROM 22, or programs that are loaded from the storage unit 25 to the RAM 23.

The RAM 23 also stores data and the like necessary for the CPU 21 to execute the various processing, as appropriate.

The communication unit 14 controls communication between the processor 11 and another apparatus (for example, the EEG apparatus 10) .

The storage unit 25 is configured by DRAM (Dynamic Random Access Memory) or the like, and stores various data managed by each server.

The input unit 26 is configured by various buttons and the like, and inputs a variety of information in accordance with instruction operations by the user.

The output unit 27 is configured by the display unit, a speaker, and the like, and outputs images and sound.

A removable medium composed of a magnetic disk, an optical disk, a magneto-optical disk, semiconductor memory or the like is installed in the drive 28, as appropriate. Programs that are read via the drive 28 from the removable medium are installed in the storage unit 25, as necessary.

In the symptom determination apparatus 20, these components operate together to perform the "process for generating reference data" and the "process for determining a symptom".

Here, the process for generating reference data is a process where the symptom determination apparatus 20 generates reference data, which is a determination reference for determining a symptom, on the basis of measurement data generated by the EEG apparatus 10.

The process for determining a symptom is a process where the symptom determination apparatus 20 determines a symptom on the basis of the measurement data generated by the EEG apparatus 10 and the reference data.

That is, in the present embodiment, the symptom determination apparatus 20 first generates reference data and then achieves an objective determination of a symptom of depression using the generated reference data.

When the process for generating reference data and the process for determining a symptom are performed, a measurement data obtaining section 211, an EEG feature detection section 212, a reference data generation section 213, a symptom determination section 214, and a determination result output section 215 function in the CPU 21 as illustrated in Fig. 3.

A measurement data storage section 251 and a reference data database 252 are provided in an area of the storage unit 25.

Although not particularly referred to in the following description, these function blocks communicates, as necessary, data necessary to achieve the processes at appropriate times.

The measurement data obtaining section 211 receives and obtains measurement data transmitted from the EEG apparatus 10. The measurement data obtaining section 211 then stores the obtained measurement data in the measurement data storage section 251. That is, the measurement data storage section 251 functions as a storage unit for storing measurement data.

The EEG feature detection section 212 detects EEG feature data, which is data indicating a feature of the measurement data stored in the measurement data storage section 251 (i.e., a feature of frequency components of the subject's EEG signal). A premise is that there are individual differences in the amplitude of an EEG signal. It is therefore not preferable to use an EEG signal as is for the determination of a symptom. The EEG feature detection section 212, therefore, normalizes the measurement data to absorb such individual differences. For example, the EEG feature detection section 212 normalizes the measurement data with a value of amplitude indicating normal distribution. As a result, the value of amplitude becomes 0 for average and 1 for variance.

Next, the EEG feature detection section 212 calculates a power spectrum indicating values of power at a plurality of frequencies (e.g., 1 to 30 [Hz]) as a plurality of frequency components of the measurement data, whose values of amplitude have been normalized, by performing a Fourier transform (e.g., a Fast Fourier transformT (FFT) employing the Hamming window) on the measurement data and averaging the measurement data. The EEG feature detection section 212 then determines the power spectrum as EEG feature data.

The EEG feature detection section 212 thus detects EEG feature data and outputs the detected EEG feature data. A destination in this case is the reference data generation section 213 during the process for generating reference data and the symptom determination section 214 during the process for determining a symptom.

The reference data generation section 213 generates reference data, which is data used by the symptom determination section 214, which will be described later, as a determination reference for determining a symptom. The reference data generation section 213, therefore, obtains evaluation data regarding a symptom relating to depression in a subject corresponding to EEG feature data used to generate the reference data. The evaluation data regarding a symptom relating to depression is not based on a determination in the process for determining a symptom but is obtained using an ordinary method based on an interview conducted by a physician, who is a determiner. The ordinary method may be one where a determination based on the Hamilton Rating Scale for Depression is made, or another method. For example, a determination based on another scale, such as Montgomery-Asberg Depression Rating Scale (MADRS) or Beck Depression Inventory (BDI), may be made, instead.

As described above, in the present embodiment, a determination whether the user U is suffering from depression and, if the user U is suffering from depression, a determination of the level of severity of a depressive symptom are made as the determination of a symptom relating to depression. The reference data generation section 213, therefore, obtains evaluation data indicating a normal person suffering from depression or, if the user U is suffering from depression, evaluation data indicating the level of severity of a depressive symptom. Any number of levels may be defined for the severity of a depressive symptom, but four levels, namely mild, moderate, severe, and most severe, for example, may be defined.

The reference data generation section 213 obtains the evaluation data regarding a symptom relating to depression on the basis of, for example, an input operation from the determiner received by the input unit 26 (or input information from the determiner received through the communication unit 14). The reference data generation section 213 constructs a database of reference data by associating the obtained evaluation data regarding a symptom relating to depression and EEG feature data regarding a corresponding subject. Here, the construction includes not only creation of a new database but also updating of an existing database with new data.

The reference data generation section 213 then stores the constructed database of reference data in the reference data database 252. That is, the reference data database 252 functions as a storage unit that stores a database of reference data.

The reference data generation section 213 thus keeps updating the database stored in the reference data database 252 by repeatedly storing EEG feature data and evaluation data in the reference data database 252 for a plurality of subjects while associating the EEG feature data and the evaluation data with each other. The database created and updated in this manner is a database indicating trends in the EEG feature data regarding the subjects corresponding to a level-wise symptom relating to depression (i.e., a normal person or one of the levels of severity).

The symptom determination section 214 determines a symptom relating to depression on the basis of the reference data in the database constructed in this manner. More specifically, the symptom determination section 214 determines a symptom relating to depression on the basis of a correlation (i.e., a relationship of intensity indicated by power spectra) between EEG feature data (i.e., a power spectrum indicating intensity of frequency components) regarding a subject of a current determination and the reference data (i.e., a power spectrum indicating intensity of frequency components) in the database constructed by the reference data generation section 213 and stored in the reference data database 252.

For example, the symptom determination section 214 compares EEG feature data regarding a subject of a current determination and the reference data in the database and identifies a piece of reference data that is most similar to the EEG feature data. The symptom determination section 214 then determines, as a determination result for the subject of the current determination, a level-wise symptom relating to depression (i.e., a normal person or a level of severity) associated, as evaluation data, with the most similar piece of reference data.

In another case, for example, the symptom determination section 214 may make a comparison for finding differences, not similarity, to make the determination. The symptom determination section 214 compares, for example, reference data associated with evaluation data indicating a normal person and EEG feature data regarding a subject of a current determination. If the reference data and the EEG feature data are not different from each other more greatly than a certain threshold, the symptom determination section 214 may determine that the subject is not suffering from depression. If the reference data and the EEG feature data are different from each other more greatly than the certain threshold, the symptom determination section 214 may determine that the subject is suffering from depression. Similarly, for example, the symptom determination section 214 compares reference data associated with evaluation data indicating a patient with depression and EEG feature data regarding a subject of a current determination. If the reference data and the EEG feature data are not different from each other more greatly than a certain threshold, the symptom determination section 214 may determine that the subject is suffering from depression. If the reference data and the EEG feature data are different from each other more greatly than the certain threshold, the symptom determination section 214 may determine that the subject is not suffering from depression.

Furthermore, after determining that a subject is suffering from depression, the symptom determination section 214 may further determine, as described above, the level of severity of a depressive symptom by identifying the most similar piece of reference data. That is, the determination whether a subject is suffering from depression and the determination of the level of severity of a depressive symptom in a case where the subject is suffering from depression may be handled as different determinations, and the determinations may be made in two steps.

Regardless of whether a comparison is made to find similarity or differences, the reference data used in the comparison may be the entirety of the reference data in the database or representative reference data generated from the entirety of the reference data in the database. For example, representative reference data regarding a normal person is generated on the basis of a representative value of all pieces of reference data associated with evaluation data indicating a normal person. In this case, the representative value is a mean, a mode, a median, or the like of the pieces of reference data. Similarly, representative reference data regarding each level of severity is generated on the basis of a representative value of all pieces of reference data associated with evaluation data indicating the level of severity. The representative reference data regarding different symptoms may then be compared with EEG feature data regarding a subject of a current determination.

A correlation of each piece of data (i.e., most similar or not, different or not) may be determined by any method. The determination may be made, for example, by comparing power spectra (i.e., frequency components) of data using a known method such as pattern matching. In this case, the power spectra may be compared at all frequencies included in the data, or just at a certain frequency. For example, the power spectra may be compared at a certain frequency at which a difference tends to occur depending on whether a subject is suffering from depression. Alternatively, the power spectra may be compared at all the frequencies while being weighted at a certain frequency to give priority to the certain frequency as a determination reference used for the comparison.

In another case, for example, the EEG feature detection section 212 may calculate not a power spectrum indicating values of power at a plurality of frequencies (e.g., 1 to 30 [Hz]) as a plurality of frequency components but a power spectrum indicating a value of power at a certain frequency (e.g., a certain frequency in 1 to 30 [Hz]) as one frequency component.

The symptom determination section 214 may then determine a symptom relating to depression on the basis of a correlation between the power spectrum indicating the intensity of the frequency component, the power spectrum being EEG feature data regarding a subject of a current determination, and the reference data in the database constructed by the reference data generation section 213 and stored in the reference data database 252.

The symptom determination section 214 thus determines a level-wise symptom relating to depression (i.e., a normal person or a level of severity) in a subject of a current determination using such a method. The symptom determination section 214 then outputs a determination result to the determination result output section 215.

The determination result output section 215 outputs, to a determiner or a subject, a determination result obtained as a result of a determination of a symptom made by the symptom determination section 214. The outputting may be, for example, display on a display included in the output unit 27, outputting of a sound from a speaker included in the output unit 27, printing onto a paper medium by a printing apparatus through the communication unit 24, or transmission, through the communication unit 24, to another apparatus (not illustrated) used by the determiner or the subject.

The symptom determination apparatus 20 can thus determine a depressive symptom in a subject on the basis of an objective indicator, namely an EEG signal in a certain brain area of the subject, and output a determination result to a determiner or the subject.

### [Measurement Process]

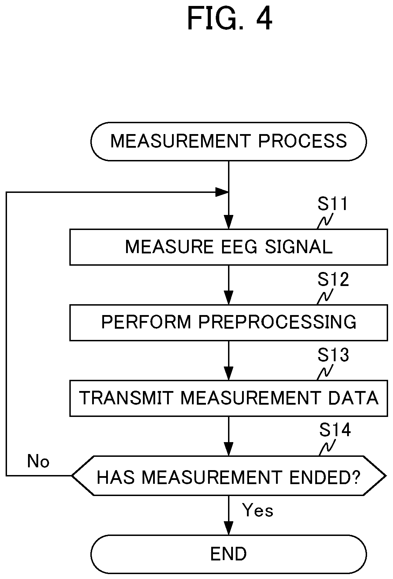

Next, how the EEG apparatus 10 performs the measurement process will be described with reference to Fig. 4. Fig. 4 is a flowchart illustrating how the EEG apparatus 10 performs the measurement process. The measurement process is performed in accordance with an operation command for starting measurement input from a subject or a determiner.

In step S11, the measurement control section 111 starts to measure the subject's EEG signal using the measuring unit 17 by controlling the measurement of the subject's EEG signal performed by the measuring unit 17. The measurement control section 111 then outputs the subject's EEG signal obtained as a result of the measurement performed by the measuring unit 17 to the preprocessing section 112.

In step S12, the preprocessing section 112 generates measurement data by preprocessing the EEG signal input from the measurement control section 111. The preprocessing section 112 then outputs the generated measurement data to the measurement data transmission section 113.

In step S13, the measurement data transmission section 113 transmits the measurement data input from the preprocessing section 112 to the symptom determination apparatus 20. Although it is assumed in the figure that the measurement data is transmitted in real-time as the measurement control section 111 generates the measurement data, the generated measurement data may be stored in the storage unit 15, and after the measurement ends, the measurement data stored in the storage unit 15 may be collectively transmitted at once, instead, as described above.

In step S14, the measurement control section 111 determines whether to end the measurement of the EEG signal performed by the measuring unit 17. When a certain period of time has elapsed since the measurement started or if an operation command for ending the measurement is input from the subject or the determiner, for example, the measurement control section 111 determines that the measurement of the EEG signal is to be ended. If the measurement of the EEG signal is to be ended, a result of step S14 is Yes, and the process ends. If the measurement of the EEG signal is not to be ended, the result of step S14 is No, and the process is repeated from step S11.

As a result of the above-described measurement process, the EEG apparatus 10 can measure a subject's EEG signal and transmit measurement data generated on the basis of the measured EEG signal to the symptom determination apparatus 20.

### [Process for Generating Reference Data]

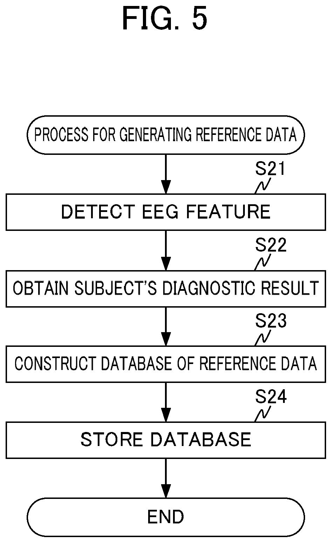

Next, how the symptom determination apparatus 20 performs a process for generating reference data will be described with reference to Fig. 5. Fig. 5 is a flowchart illustrating how the symptom determination apparatus 20 performs the process for generating reference data. The process for generating reference data is performed when an operation command for starting to generate reference data is input from a determiner or a manager of the symptom determination system S. A premise of the process is that measurement data generated as a result of the measurement process has been received by the measurement data obtaining section 211 and stored in the measurement data storage section 251.

In step S21, the EEG feature detection section 212 detects EEG feature data from the measurement data stored in the measurement data storage section 251.

In step S22, the reference data generation section 213 obtains evaluation data regarding a determination of a symptom relating to depression in a subject corresponding to the EEG feature data used to create reference data.

In step S23, the reference data generation section 213 constructs a database of reference data by associating the obtained evaluation data with the corresponding subject's EEG feature data. As described above, the construction includes not only creation of a new database but also updating of an existing database with new data.

In step S24, the reference data generation section 213 stores the constructed database of reference data in the reference data database 252. The process thus ends.

As a result of the above-described process for generating reference data, the symptom determination apparatus 20 can generate reference data, which is a determination reference for the symptom determination section 214 to make a determination of a symptom.

### [Process for Determining Symptom]

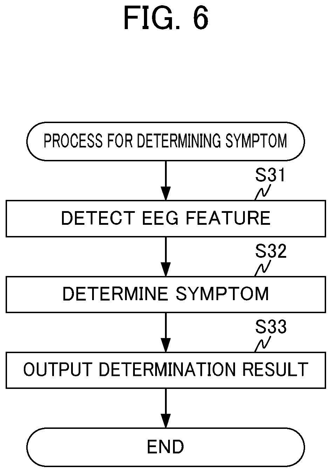

Next, how the symptom determination apparatus 20 performs a process for determining a symptom will be described with reference to Fig. 6. Fig. 6 is a flowchart illustrating how the symptom determination apparatus 20 performs the process for determining a symptom. The process for determining a symptom is performed when an operation command for starting a determination of a symptom input from a determiner or the like. A premise of the process is that measurement data generated as a result of the measurement process has been received by the measurement data obtaining section 211 and stored in the measurement data storage section 251. A database of reference data constructed as a result of the process for generating reference data is stored in the reference data database 252.

In step S31, the EEG feature detection section 212 detects EEG feature data from the measurement data stored in the measurement data storage section 251.

In step S32, the symptom determination section 214 makes a determination of a symptom relating to depression on the basis of a subject's EEG feature data in step S32 and the reference data in the database constructed as a result of the above-described process for generating reference data.

In step S33, the determination result output section 215 outputs a result of the determination made by the symptom determination section 214 in step S32.

As a result of the above-described process for determining a symptom, the symptom determination apparatus 20 determines a depressive symptom in a subject on the basis of an objective indicator, namely an EEG signal in a certain brain area of the subject.

With the symptom determination system S according to the present embodiment, therefore, a symptom of a mental disorder can be objectively determined independently of a determiner's subjectivity.

In addition, with the process for determining a symptom, the general problem of the related art can be solved since a symptom of a mental disorder is objectively determined. The above-described problem that a result of a determination inevitably reflects a determiner's subjectivity when the determiner takes the lead in determining a symptom, for example, can be solved.

### [Example]

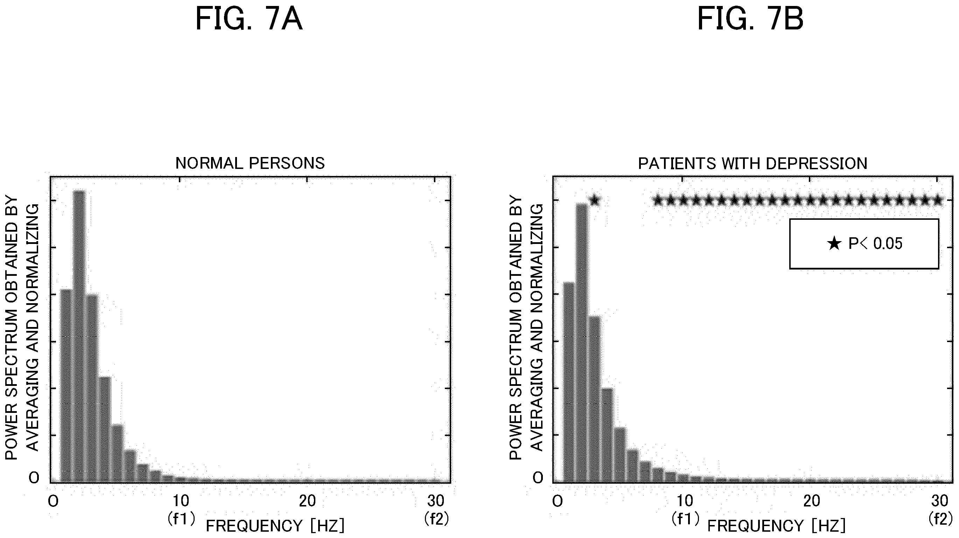

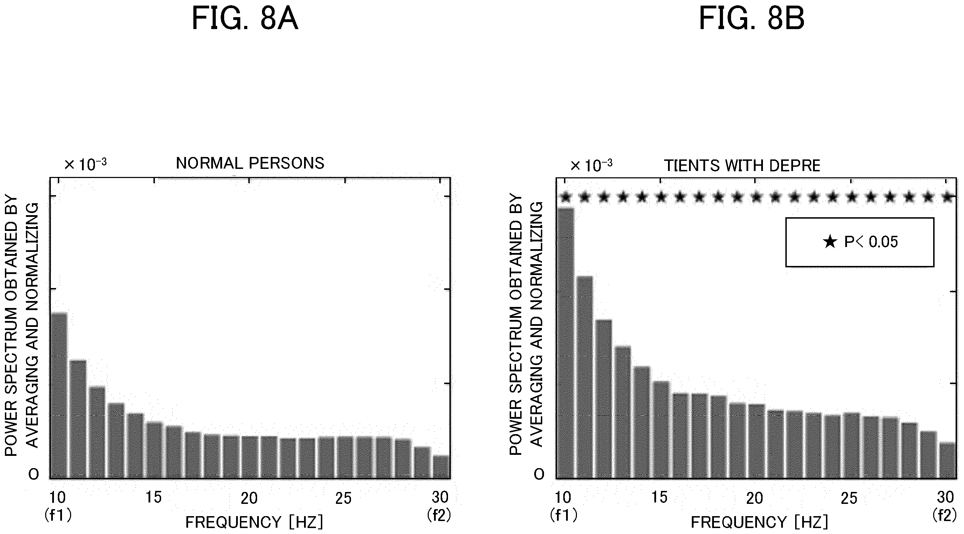

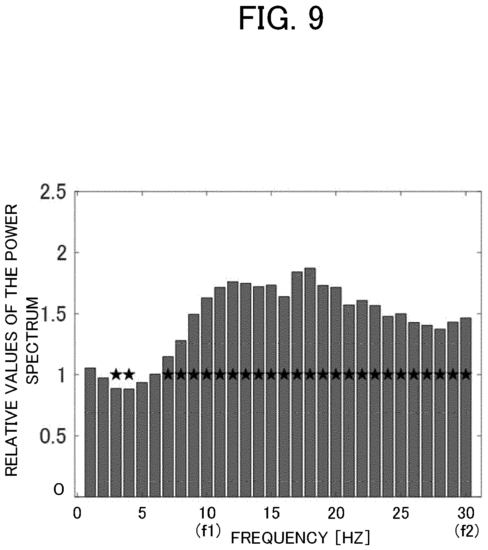

An embodiment of the present invention has been described. Next, an example of the embodiment of the present invention will be described with reference to Figs. 7, 8, and 9. Here, Fig. 7 includes graphs illustrating a result of comparison of EEG features between normal persons and patients with depression in this example. Fig. 8 includes graphs obtained by enlarging the result of comparison of EEG signals between the normal persons and the patients with depression in this example. Fig. 9 is a graph illustrating the result of comparison of EEG features between the normal persons and the patients with depression in this example using relative values.

First, Fig. 7(a) illustrates a power spectrum obtained by averaging and normalizing EEG feature data regarding a plurality of normal persons. Similarly, Fig. 7(b) illustrates a power spectrum obtained by averaging and normalizing EEG feature data regarding a plurality of patients with depression.

In this example, a two-sample t-test was conducted on each of frequencies as a test of significance with a frequency of the normal persons set at 1 (p < 0.05). Asterisks (i.e., star marks) in Fig. 7(b) indicate frequencies significantly different between the normal persons and the patients with depression. Figs. 8(a) and 8(b) illustrate an enlarged high-frequency range (a frequency range corresponding to frequencies f1 to f2 (e.g., 10 to 30 [Hz] here) in the figures), where especially significant differences were observed. The power spectra were thus more significantly different from each other in the high-frequency range than in a low-frequency range.

In order to prove that such differences indicate differences between the normal persons and the patients with depression, the normal persons were also compared with one another through a two-sample t-test. No significant differences, however, were observed (p < 0.05), and results were the same even when significant differences were examined with higher critical p-values.

It is therefore obvious from a result of this example illustrated in Fig. 7(b) that the EEG feature data (i.e., the power spectrum indicating intensity of frequency components) regarding the normal persons and the EEG feature data (i.e., the power spectrum indicating intensity of frequency components) regarding the patients with depression are significantly different from each other. The symptom determination section 214, therefore, can accurately determine a symptom relating to depression using the above-described method.

Fig. 9 is a diagram illustrating relative values (i.e., values obtained by dividing the power spectrum of the patients with depression with that of the normal persons at each of frequencies) of the power spectrum of the patients with depression illustrated in Fig. 7(b) at a time when the power spectrum of the normal persons illustrated in Fig. 7(a) is set at 1. In Fig. 9, the closer the relative value to 1, the similar the patients with depression to the normal persons. It can also be seen from this figure that the power spectra were more significantly different from each other in the high-frequency range than in the low-frequency range. In view of this, the symptom determination section 214 may weight a power spectrum in the low-frequency range to give priority to the power spectrum as a determination reference used for comparison.

Although values of the frequencies f1 and f2, which are a lower limit and an upper limit of the high-frequency range are 10 [Hz] and 30 [Hz], respectively, in the above-description with reference to Figs. 7, 8, and 9, this is just a preferred example, and is not intended to limit the values of the frequencies f1 and f2.

### [Modifications]

Although an embodiment of the present invention has been described, the embodiment is just an example, and does not limit the technical scope of the present invention. The present invention can take various other embodiments without deviating from the spirit thereof and be subjected to various types of modification such as omission and replacement. In these cases, such embodiments and modifications are included in the scope and spirit of the invention described herein and the scope of the invention described in the claims and its equivalent scope.

As an example, the above-described embodiment of the present invention may be modified as in the following modifications.

### <First Modification>

In the above-described embodiment, the symptom determination section 214 determines a symptom relating to depression on the basis of a correlation (i.e., a relationship of intensity) between EEG feature data (i.e., a power spectrum indicating intensity of frequency components) regarding a subject of a current determination and the reference data (i.e., a power spectrum indicating intensity of frequency components) in the database constructed by the reference data generation section 213 and stored in the reference data database 252. The symptom determination section 214, however, may make the determination using other methods, instead. For example, as described above as an example with reference to the drawings, power spectra are more significantly different from each other in the high-frequency range than in the low-frequency range.

That is, a power spectrum of a patient with depression is equal to or lower than that of a normal person in the low-frequency range, but higher than that of a normal person in the high-frequency range. That is, a relative relationship between the high-frequency range and the low-frequency range of a power spectrum is different between a patient with depression and a normal person.

Whether a person is a patient with depression or a normal person can be determined on the basis of this point of view by examining a relative relationship between the low-frequency range and the high-frequency range of a power spectrum in EEG feature data regarding one subject. According to the present modification, therefore, whether a person is a patient with depression or a normal person can be determined without performing comparison with reference data as in the above-described embodiment.

### <Second Modification>

In another modification of the above-described embodiment, an effect of a medicine taken by a patient with depression may be taken into consideration. Patients with depression usually take benzodiazepines, antidepressants, anxiolytics, or other medicines for purposes such as suppression of symptoms of depression.

Since actions are different between these medicines, EEG feature data regarding a subject is differently affected depending on the medicine (i.e., an effect upon a power spectrum). How an effect of each medicine upon EEG feature data is different, therefore, is statistically examined in advance by comparing EEG feature data between patients with depression of the same level of severity who take or do not take the medicine (or before and after the same person takes the medicine).

If a subject of a current determination takes any medicine, the symptom determination section 214 corrects EEG feature data on the basis of statistical data obtained in advance. That is, the symptom determination section 214 corrects a power spectrum in such a way as to offset an effect of the medicine upon the power spectrum. The symptom determination section 214 then determines a symptom relating to depression as in the above-described embodiment on the basis of the corrected power spectrum. A symptom relating to depression can thus be determined more accurately while eliminating an effect of each medicine.

### <Other Modifications>

Although a unipolar EEG signal at Fp1 is measured in the above-described embodiment, EEG signals from a plurality of sources may be measured, instead. A symptom relating to depression may then be determined on the basis of each of the EEG signals. In this case, for example, a symptom relating to depression may be determined as described above for each of the EEG signals measured from the plurality of sources, and an average of results of the determinations may be used as a determination result for the user U.

In addition, although the EEG apparatus 10 and the symptom determination apparatus 20 are implemented as separate apparatuses in the above embodiment, the EEG apparatus 10 and the symptom determination apparatus 20 may be integrated together as a single apparatus, instead.

### [Configuration Examples]

As described above, the symptom determination apparatus 20 according to the present embodiment includes the EEG feature detection section 212 and the symptom determination section 214.

The EEG feature detection section 212 detects one or a plurality of frequency components of an EEG signal in a certain brain area of a subject.

The symptom determination section 214 determines a depressive symptom in the subject on the basis of intensity of the one or plurality of frequency components of the EEG signal detected by the EEG feature detection section 212.

The symptom determination apparatus 20 thus determines a depressive symptom in a subject on the basis of an objective indicator, namely an EEG signal in a certain brain area of the subject.

With the symptom determination apparatus 20, therefore, a symptom relating to a mental disorder can be objectively determined independently of a determiner's subjectivity.

The symptom determination section 214 determines a depressive symptom in the subject on the basis of a correlation between the one or plurality of frequency components of the EEG signal detected by the EEG feature detection section 212 and the one or plurality of frequency components of an EEG signal that serves as a reference for determining a depressive symptom.

As a result, the symptom determination apparatus 20 can determine a symptom relating to a mental disorder more objectively on the basis of a certain reference.

The EEG feature detection section 212 detects the plurality of frequency components of the EEG signal, and
the symptom determination section 214 determines the depressive symptom while giving priority, as a determination reference, to a relationship of intensity of, among the plurality of frequency components of the EEG signal detected by the EEG feature detection section 212, a frequency component at a certain frequency over relationships of intensity of the frequency components at other frequencies.

As a result, a symptom relating to a mental disorder can be determined while giving priority, as a determination reference, to a relationship of intensity of a frequency component at a certain frequency suitable as an indicator for determining a depressive symptom.

The EEG feature detection section 212 detects the plurality of frequency components of the EEG signal, and
the symptom determination section 214 determines the depressive symptom in the subject on the basis of a result of comparison of intensity between, among the plurality of frequency components of the EEG signal detected by the EEG feature detection section 212, a frequency component in a low-frequency range and a frequency component in a high-frequency range.

As a result, the symptom determination apparatus 20 can objectively determine a symptom relating to a mental disorder without using an EEG signal of a person other than a subject.

The symptom determination section 214 determines a level of severity of the depressive symptom in the subject.

As a result, not only a determination whether a subject is suffering from depression but also, if the subject is suffering from depression, a determination of a level of severity of a depressive symptom can be made.

The symptom determination section 214 corrects, if the subject takes a medicine, a determination reference for determining the depressive symptom in the subject on a basis of an effect of the medicine taken by the subject.

As a result, a determination can be made more accurately in consideration of an effect of a medicine taken by a subject.

### [Function Fulfilled by Hardware or Software]

The functions for performing the series of processes according to the embodiment described above can be fulfilled by using hardware, can be fulfilled by using software, or can be fulfilled by using a combination thereof. In other words, an aspect to fulfill the functions is not particularly limited provided that the functions for performing the series of processes described above are fulfilled by the SYMPTOM DETERMINATION SYSTEM S.

For example, in the case where the functions for performing the series of processes described above are fulfilled by a processor that performs an arithmetic operation, examples of the processor that performs the arithmetic operation include processing apparatus such as a single processor, multiple processors, or a multi-core processor, and a combination of the processing apparatus and a processing circuit such as an ASIC (Application Specific Integrated Circuit) or a FPGA (Field-Programmable Gate Array).

For example, in the case where the functions for performing the series of processes described above are fulfilled by using software, a program that is included in the software is install in a computer via a network or a recording medium. In this case, the computer may be a computer into which exclusive hardware is incorporated or may be a general-purpose computer (for example, a typical electronic device such as a general-purpose personal computer) that can perform a predetermined function by installing a program. Steps of a written program may include only processes that are sequentially performed in time series and may include processes that are performed in parallel or processes that are separately performed. The steps of the written program may be performed in a freely selected order without departing from the spirit of the present invention.

The recording medium in which the program is recorded may be provided to a user by being distributed separately from the computer or may be provided to a user with the recording medium incorporated into the computer in advance. In this case, examples of the recording medium that is distributed separately from the computer include a magnetic disk (including a floppy disk), an optical disk, and a magneto-optical disk. Examples of the optical disk include a CD-ROM (Compact Disc-Read Only Memory), a DVD (Digital Versatile Disc), and a Blu-ray (register trademark) Disc (a Blu-ray Disc). Examples of the magneto-optical disk include a MD (Mini Disc) . Examples of the recording medium that is provided to a user with the recording medium incorporated into the computer in advance include the ROM 12 in Fig. 2, the ROM 22 in Fig. 3, the storage unit 15 in Fig. 2, or the storage unit 25 in Fig. 3, in which the program is recorded.

### [Reference Signs List]

10 electroencephalography (EEG) apparatus, 20 symptom determination apparatus, 11,21 CPU, 12,22 ROM, 13,23 RAM, 14,24 communication unit, 15,25 storage unit, 16,26 input unit, 17 measuring unit, 27 output unit, 28 drive, 111 measurement control section, 112 preprocessing section, 113 measurement data transmission section, 211 measurement data obtaining section, 212 EEG feature detection section, 213 reference data generation section, 214 symptom determination section, 215 determination result output section, 251 measurement data storage section, 252 reference data database, S symptom determination system, U user

## Claims

1. A symptom determination apparatus comprising:
electroencephalography signal detection means for detecting one or a plurality of frequency components of an electroencephalography signal in a certain brain area of a subject; and
depressive symptom determination means for determining a depressive symptom in the subject on a basis of intensity of the one or plurality of frequency components of the electroencephalography signal detected by the electroencephalography signal detection means.

2. The symptom determination apparatus according to claim 1,
wherein the depressive symptom determination means determines a depressive symptom in the subject on a basis of a correlation between the one or plurality of frequency components of the electroencephalography signal detected by the electroencephalography signal detection means and the one or plurality of frequency components of an electroencephalography signal that serves as a reference for determining a depressive symptom.

3. The symptom determination apparatus according to claim 1 or 2,
wherein the electroencephalography signal detection means detects the plurality of frequency components of the electroencephalography signal, and
wherein the depressive symptom determination means determines the depressive symptom while giving priority, as a determination reference, to a relationship of intensity of, among the plurality of frequency components of the electroencephalography signal detected by the electroencephalography signal detection means, a frequency component at a certain frequency over relationships of intensity of the frequency components at other frequencies.

4. The symptom determination apparatus according to any of claims 1 to 3,
wherein the electroencephalography signal detection means detects the plurality of frequency components of the electroencephalography signal, and
wherein the depressive symptom determination means determines the depressive symptom in the subject on a basis of a result of comparison of intensity between, among the plurality of frequency components of the electroencephalography signal detected by the electroencephalography signal detection means, a frequency component in a low-frequency range and a frequency component in a high-frequency range.

5. The symptom determination apparatus according to any of claims 1 to 4,
wherein the depressive symptom determination means determines a level of severity of the depressive symptom in the subject.

6. The symptom determination apparatus according to any of claims 1 to 5,
wherein the depressive symptom determination means corrects, if the subject takes a medicine, a determination reference for determining the depressive symptom in the subject on a basis of an effect of the medicine taken by the subject.

7. A program for determining a symptom, the program causing a computer to achieve:
an electroencephalography signal detection function for detecting one or a plurality of frequency components of an electroencephalography signal in a certain brain area of a subject; and
a depressive symptom determination function for determining a depressive symptom in the subject on a basis of intensity of the one or plurality of frequency components of the electroencephalography signal detected by the electroencephalography signal detection function.
